# EUROPEAN PATENT SPECIFICATION

(11) **EP 0 543 107 B1**
(45) Date of publication and mention of the grant of the patent: **03.12.1997**
(21) Application number: 92115989.3
(22) Date of filing: 18.09.1992
(51) Int. Cl.: A61B 17/00, A61B 17/28

(54) **Endoscopic surgical instruments and jaw structure**
Endoskopische chirurgische Instrumente und Backenstruktur
Instruments chirurgicaux endoscopiques et structure de machoires

(30) Priority: 18.10.1991 US 781069
(43) Date of publication of application: 26.05.1993
(73) Proprietor: United States Surgical Corporation, Norwalk, Connecticut 06856 (US)
(72) Inventor: Aranyi, Ernie, Easton, CT 06612 (US)
(74) Representative: Marsh, Roy David

(56) References cited:
- EP-A- 0 023 526
- EP-A- 0 065 054
- DE-U- 9 106 506
- GB-A- 973 059
- GB-A- 2 044 108
- US-A- 3 746 002
- US-A- 4 043 323
- US-A- 4 655 219

## Description

The present invention relates to surgical instruments. The invention is applicable to handle and jaw structures for a endoscopic or laparoscopic surgical instrument having reciprocating jaw members which pivot in response to the opening and closing of the handle members, where movement of the handle members is translated through an elongated tubular body member to open and close the jaw mechanism.

In the prior art, various endoscopic surgical instruments are disclosed which utilize generally complex mechanisms for opening and closing handle members and jaw members to facilitate use of the device at a surgical site. Many devices provide an intricate construction in which a linkage mechanism for opening and closing the jaws requires numerous moving parts, while a sliding arrangement is provided between two extended rod members to activate the linkage mechanism in response to movement of the handle members. In addition, pivoting of the handle members in many cases causes an unwanted radial torquing force on the rod which requires additional space to be provided in the handle members to accommodate the radial movement of the rod.

Furthermore, it is often necessary for the surgeon, or an assistant, to maintain a constant force on the handles to keep the jaw mechanism closed in the event that the instrument is a grasping or gripping device such as forceps, needle holders, or retractors. This limits the surgeon's range, and in the case of an assistant, requires additional personnel to be present in the operating room, thus restricting movement in an already confining location. To alleviate this problem, it has been known to provide locking mechanisms on the handles of the surgical instruments which allow the surgeon to lock or clamp the jaw members in place to free his hands to operate additional instruments during the course of the operation. This frees the surgical assistant to support the surgeon and eliminates the need for additional assistants. Typical locking devices include arm members which extend between the handles so that a series of ridges or ribs on each arm member engage corresponding ridges on the opposite arm to lock the handles in position. Bending one arm in relation to the other releases the locking mechanism.

A disadvantage associated with these devices concerns the release of the locking mechanism for subsequent movement of the jaw members to remove or reposition the instrument. Generally, the arm members of locking mechanisms are constructed of a resilient material, such as stainless steel or rigid plastic, and the locking forces which hold the arm members in engagement are generated by the natural flexing and biasing of the material of which the arm members are constructed. To release the locking mechanism, the arms must be disengaged by overcoming the locking forces of the arms. Typically, this is accomplished by manually flexing the arms away from each other, necessitating the use of two hands, one to grasp the instrument, and the other to forcibly move the arm members. This, of course, requires the surgeon (or assistant) to cease what he is doing and release the mechanism, thus reducing the effectiveness of the surgeon during the operation, particularly in an emergency situation.

A further disadvantage lies in the fact that typical locking mechanisms cannot be overridden; that is, the mechanism is always engaged, thereby preventing free movement of the handle and jaw mechanism. This usually requires the surgeon to choose an instrument either having the locking mechanism or one that does not. This leads to an over-abundance of instruments in the operating room and tends to complicate an already complex situation.

Finally, locking mechanisms located on the handles require special care in sterilization, packaging and storage, as well as in normal handling in the operating room. Dirt and debris may clog the ribs of the locking mechanism thus reducing its effectiveness, and damage to the ribs during storage or packaging may destroy the ribs, rendering the locking mechanism useless.

U.S. Patent No. 1,452,373 to Gomez discloses a typical locking mechanism for a surgical instrument, in which a plurality of ribs are provided on an extension of the handle member which engage a similar rib member on the opposite handle. Once engaged, the handles must be moved away from each other perpendicular to their longitudinal axis to disengage the locking mechanism to release the jaw mechanism.

U.S. Patent No. 4,896,661 to Bogert et al. disclose a surgical instrument having a ratchet mechanism positioned on the handle members which includes a curved rack member attached to one handle member which passes through a slot in the other handle member. A releasable pawl member is provided on the second handle to engage the rack member and provide a means for releasing the ratchet.

U.S. Patent No. 4,935,027 to Yoon discloses a surgical instrument having a ratchet mechanism positioned between the handle members. A rack member is provided which extends from one handle and passes through a slot in the second handle to lock the handles in place. Pivoting the rack member away from corresponding grooves in the slot will release the ratchet mechanism.

U.S. Patent No. 4,428,374 to Auburn discloses a surgical instrument having means for positioning and holding the handle members in relation to each other. A rack member is provided on one handle member which extends through a slot in the second handle member in which a releasable pawl mechanism is provided to engage and disengage the ratcheting mechanism.

With respect to jaw mechanisms and, in particular, atraumatic jaw mechanisms such as graspers and clamps, it is important for surgeons to be able to gauge or "feel" the amount of force being applied to the jaw mechanisms. This ability is particularly important in endoscopic procedures where visibility is somewhat limited and surgeons must place a greater reliance on their sense of touch. In conventional jaw mechanisms, the elements of the jaws are typically forged or cast of a rigid material into a predetermined shape. These forged or cast elements require elaborate metal working facilities and a relatively large amount of labor in finishing the elements. The finished elements usually exhibit very little flexural ability thus inhibiting the surgeon's perception of the amount of force to which the grasped or clamped tissue is exposed.

A further example of prior art instruments is given in US-A-4 043 323.

The novel surgical instrument pursuant to the present invention obviates the disadvantages encountered in the prior art and provides a precise instrument which is easy to manufacture and efficient to use, which eliminates many of the moving parts required by prior art devices. The instrument of the present invention incorporates many features which are of use to the surgeon during an operation, in particular may include an internal ratcheting mechanism to provide for incremental movement of the tool mechanism and locking of the jaws if desired, while maintaining a lightweight construction in an easy to handle device in which all of the features may be operated with one hand. Also, the features are so positioned so as to provide a maximum line of sight for the surgeon without obstructing the view to the surgical site. Furthermore, novel jaw mechanisms may be incorporated which are easy to precisely manufacture and, in the case of atraumatic graspers or clamps, have flexural capabilities which allow surgeons to gauge the amount of force being applied.
It is an object of the present invention to provide jaw mechanisms which enable users to gauge the degree of force applied to the jaw members or to tissue captured therebetween.

According to the invention, there is provided a surgical instrument comprising: a pair of jaws, each jaw formed from a single sheet of material having a proximal, distal and intermediate portion, said jaws being twisted in said intermediate portion such that said distal portion is substantially perpendicular to said proximal portion; means for pivotally mounting said jaws in co-operative alignment; and means for selectively pivoting said jaws, wherein said jaws are precambered such that when they are pivoted to a closed position, said jaws contact each other at respective contact surfaces of the distal portions thereof before said intermediate portions come into contact.

The foregoing objects and other features of the invention will become more readily apparent and may be understood by referring to the following detailed description of an illustrative embodiment of the endoscopic or laparoscopic surgical instrument having an internal ratchet mechanism, taken in conjunction with the accompanying drawings, in which:
Figure 1 illustrates a perspective view of an endoscopic or laparoscopic surgical instrument possessing the ratchet mechanism according to a first explanatory embodiment;
Figure 2 illustrates a side plan view and partial cut-away of the surgical instrument of Figure 1;
Figure 3 illustrates an exploded side cut-away view of the device of Figure 2 showing in detail the ratchet mechanism of the instrument of Figure 1;
Figures 4 and 5 illustrate a side plan view and a perspective view, respectively, of the ratchet mechanism of the explanatory embodiment of Figure 1;
Figure 6 illustrates a perspective view of an alternate explanatory embodiment of the endoscopic or laparoscopic surgical instrument employing a ratchet mechanism;
Figure 7 illustrates a side plan view of the laparoscopic surgical instrument of Figure 6;

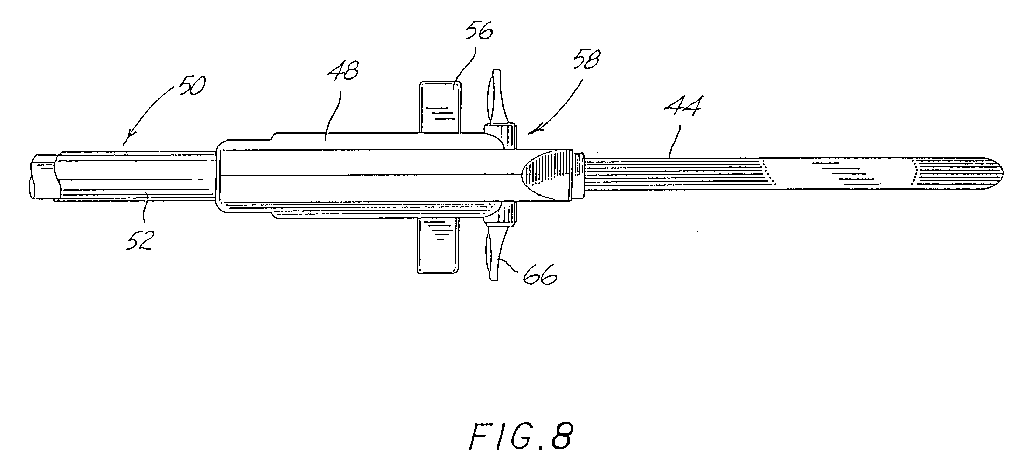

Figure 8 illustrates a top plan view of the device of Figure 6;
Figure 9 illustrates a side cut-away view of the endoscopic or laparoscopic surgical instrument of Figure 6 having a ratchet mechanism;
Figure 10 illustrates a further explanatory embodiment of the endoscopic or laparoscopic surgical instrument employing a ratchet mechanism;

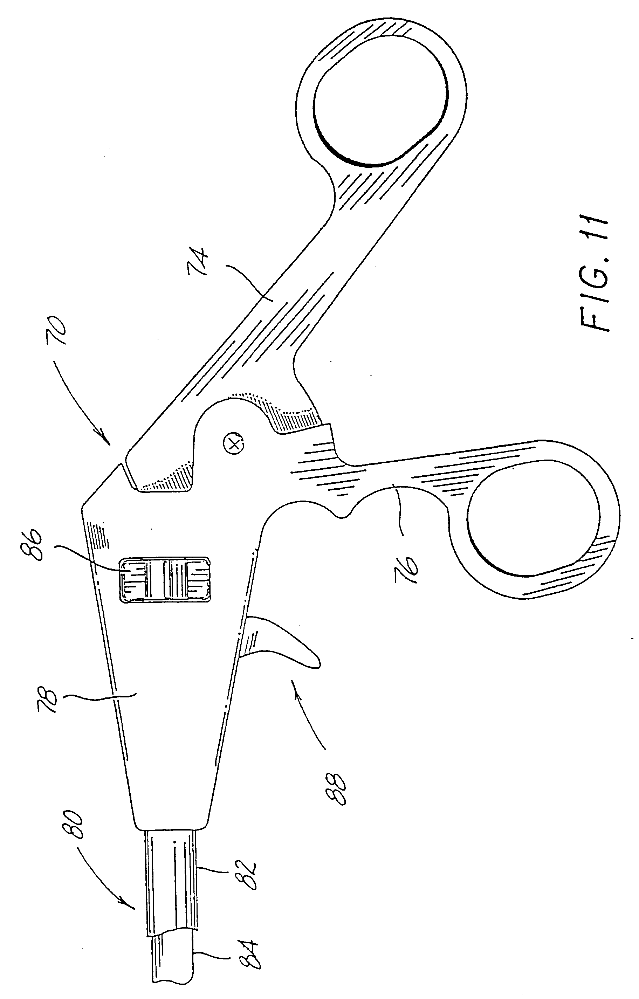

Figure 11 illustrates a side view of the device of Figure 10;
Figure 12 illustrates a side cut-away view of the device of Figure 10;
Figure 13 illustrates another explanatory embodiment of the endoscopic or laparoscopic surgical instrument employing a ratchet mechanism;
Figure 14 illustrates a side plan view of the device of Figure 13;
Figure 15 illustrates a side cut-away view of the device of Figure 13;
Figure 16 illustrates a perspective view of a further explanatory embodiment of the endoscopic or laparoscopic surgical instrument employing a ratchet mechanism;

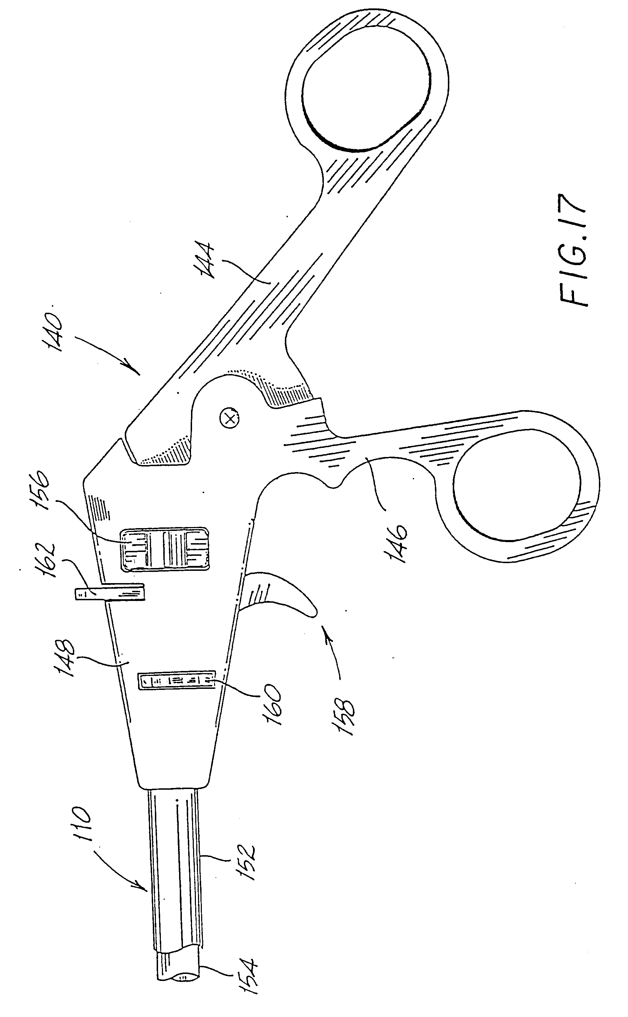

Figure 17 illustrates a side plan view of the device of Figure 16;
Figure 18 illustrates a side cut-away view of the device of Figure 16;
Figure 19 illustrates a plan view of an embodiment of an actuation means for use with a ratchet mechanism;
Figure 20 illustrates a plan view of a stop means for use with a rotation knob;
Figure 21 illustrates a top plan view of a jaw member in accordance with one embodiment of the present invention prior to being formed;
Figure 22 illustrates a top plan view of the jaw member of Figure 21 wherein a proximal end is bent relative to the distal end;
Figure 23 illustrates a side plan view of the jaw member of Figure 22 wherein the distal end is formed in a semicircular configuration;
Figure 24 illustrates a perspective view in partial phantom of a jaw mechanism in accordance with one embodiment of the present invention with the jaw members closed;
Figure 25 illustrates a perspective view of the jaw mechanism of Figure 24 with the jaw members in the open position;
Figure 26 illustrates a perspective view of an endoscopic instrument incorporating the jaw mechanism of Figure 24;
Figure 27 illustrates a top plan view of a jaw member in accordance with another embodiment of the present invention;
Figure 28 illustrates a front plan view in cross section of a jaw member taken along line 28-28 of Figure 27;
Figure 29 illustrates a side plan view of the jaw member of Figure 27;
Figure 30 illustrates a perspective view in partial phantom of a jaw mechanism in accordance with jaw members of Figure 27 in the closed position;
Figure 31 illustrates a perspective view of the jaw mechanism of Figure 30 with the jaw members in the open position;
Figure 32 illustrates a perspective view of a jaw member in accordance with another embodiment of the present invention; and
Figure 33 illustrates a side plan view in cross section of an endoscopic instrument incorporating the jaw member of Figure 32.

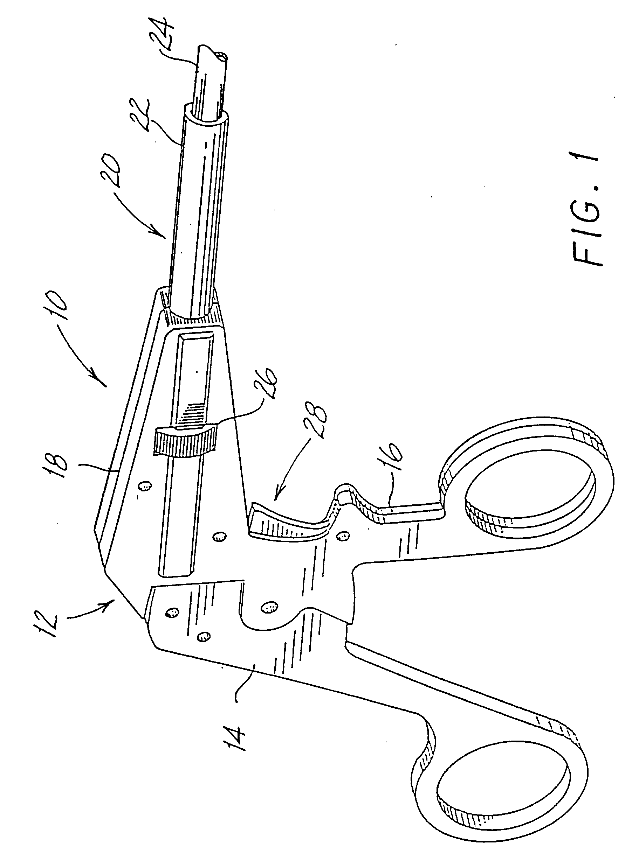

Referring now in specific detail to the drawings, in which like reference numerals identify similar or identical elements, Figure 1 illustrates a first explanatory embodiment a handle of an endoscopic or laparoscopic surgical instrument 10. In its simplest form, device 10 comprises a handle assembly 12, a body assembly 20, and a ratchet mechanism 28. Handle assembly 12 comprises a pivoting handle 14, a stationary handle 16, and a barrel portion 18 to which body assembly 20 is attached. Body assembly 20 essentially comprises an outer tubular member 22 through which an inner rod member 24 coaxially passes in a slidable arrangement. Preferably, outer tube 22 is secured to barrel portion 18 and remains stationary during operation of the device. Upon movement of pivoting handle 14, inner rod 24 reciprocates within tube member 22 to operate a tool mechanism provided at the distal end of the instrument 10. This tool mechanism (not shown) may comprise a surgical implement, such as scissors, graspers, forceps, retractors and the like. A rotation knob 26 may be provided which rotates body assembly 20 to orient the tool mechanism at various angles to the longitudinal axis.

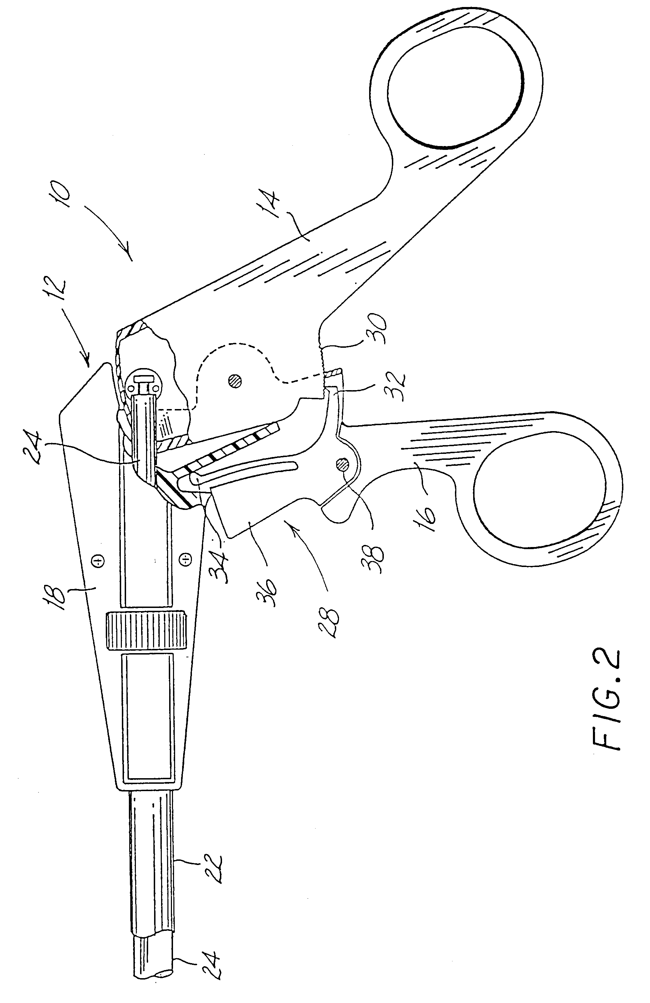

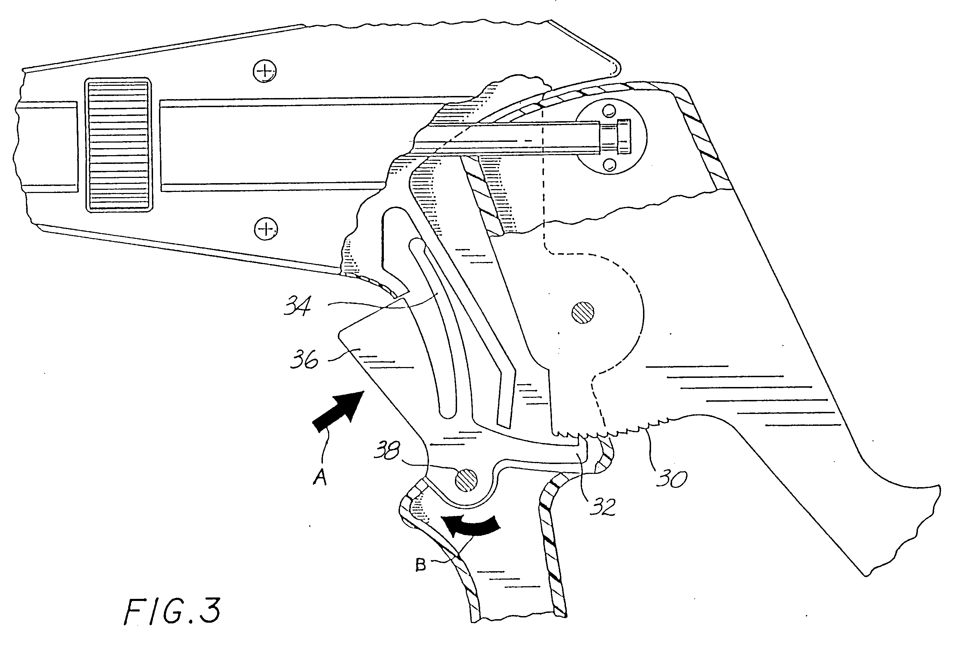

As best seen in Figures 2 and 3, ratchet mechanism 28 is provided to incrementally adjust and hold the position of pivoting handle 14. This incremental positioning, which sets pivoting handle 14 at various locations along its path of travel, provides a means to incrementally open and close the tool mechanism during the surgical procedure. Ratchet mechanism 28 is essentially positioned internally within barrel portion 18 and stationary handle 16 so that none of the mechanism is exposed to environmental conditions.

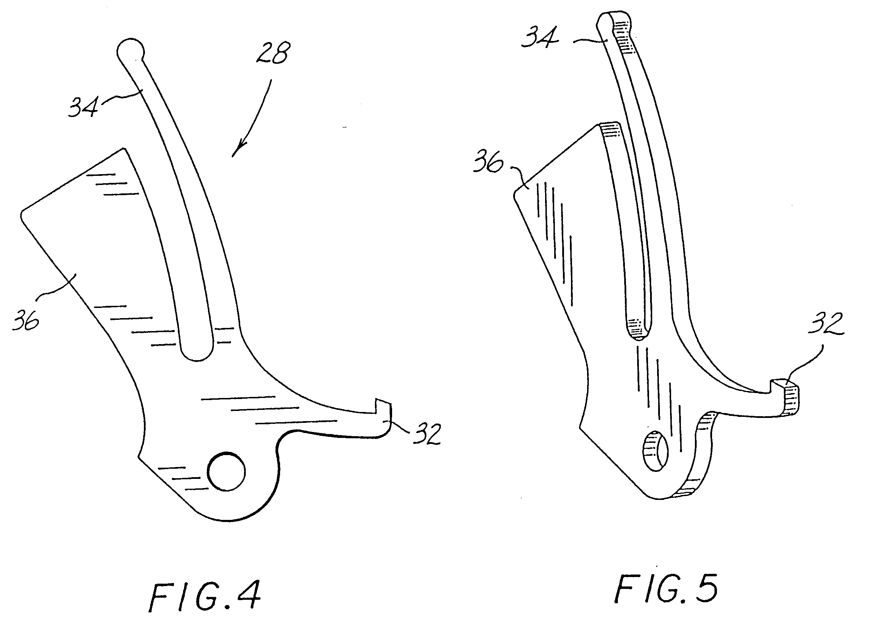

Ratchet mechanism 28 includes a pawl member 32 which engages a rack member 30 which is located on the pivoting handle 14. Rack member 30 comprises a plurality of indentations or notches which accepts pawl arm 32 to hold pivoting arm 14 in place. Ratchet mechanism 28 utilizes an integrally constructed leaf spring member 34 which biases ratchet mechanism 28 into the engaged position such that pawl member 32 engages rack member 30. To release the ratchet mechanism, a trigger member 36 is provided which, when depressed by the user in the direction of arrow A in Figure 3, causes the ratchet mechanism 28 to pivot about pivot point 38 in the direction of arrow B to disengage pawl member 32 from rack member 30. It can be appreciated that continual depression of trigger member 36 in the direction of arrow A allows the ratchet mechanism 28 to be overridden so that pivoting handle 14 may operate freely without the constraints of ratchet mechanism 28. Releasing trigger member 36 will return the pawl member 32 to the engaged position. Ratchet mechanism 28 can be best seen in Figures 4 and 5.

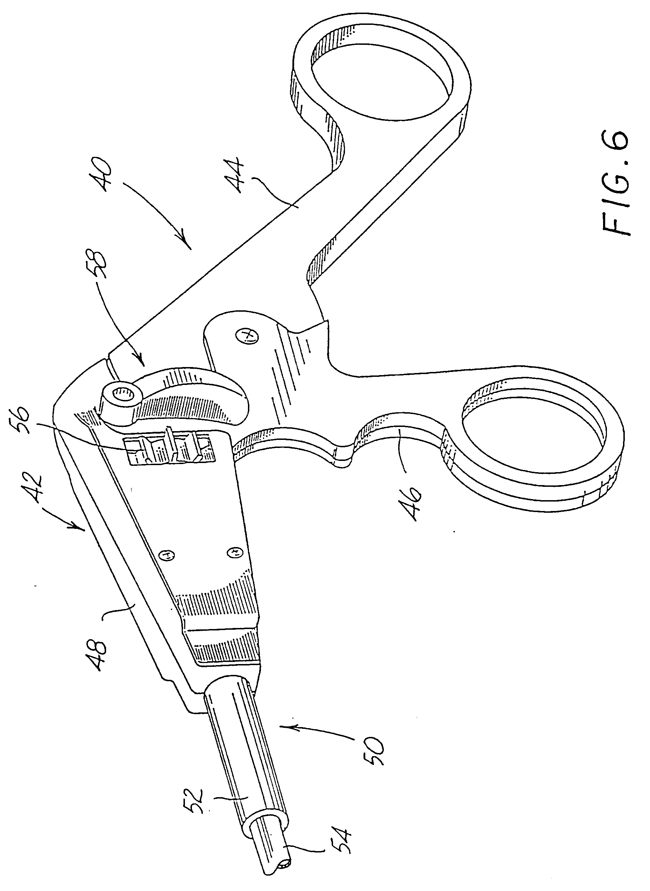

Figure 6 illustrates a second explanatory embodiment of a surgical instrument employing a ratchet mechanism as described above. Instrument 40 is similar to instrument 10 described above and includes a handle portion 42 to which body assembly 50 is attached. Body assembly 50 terminates in a tool mechanism similar to that described above.

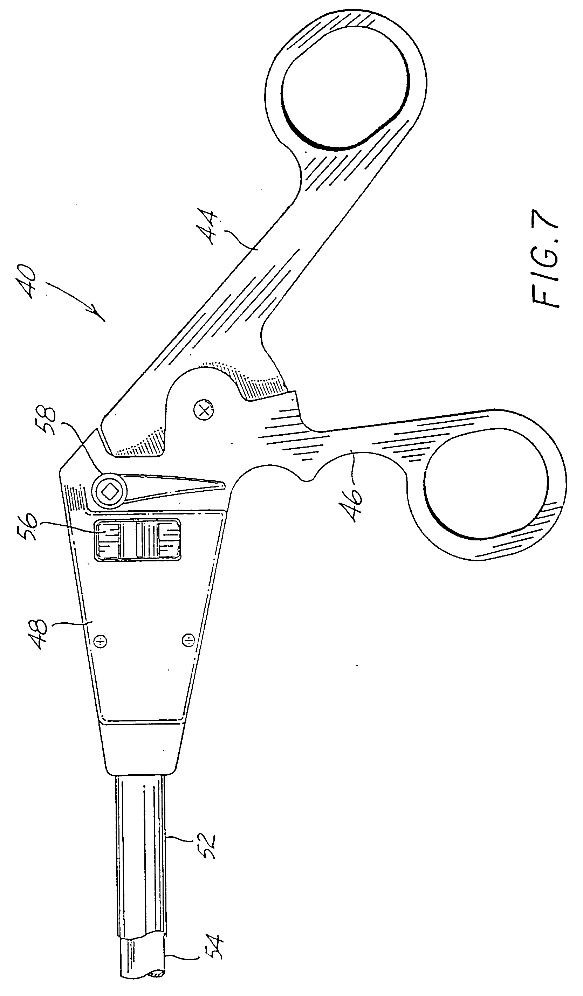

Handle assembly 42 comprises a pivoting handle 44, a stationary handle 46 and a barrel portion 48. Body assembly 50 comprises an outer tubular member 52 through which an inner rod member 54 coaxially passes in sliding arrangement. Movement of pivoting handle 44 causes inner rod member 54 to reciprocate within outer tube 52. Outer tube 52 is secured within barrel portion 48. As can be seen in Figure 6 and Figure 7, a rotation knob 56 may be provided, along with ratchet mechanism 58.

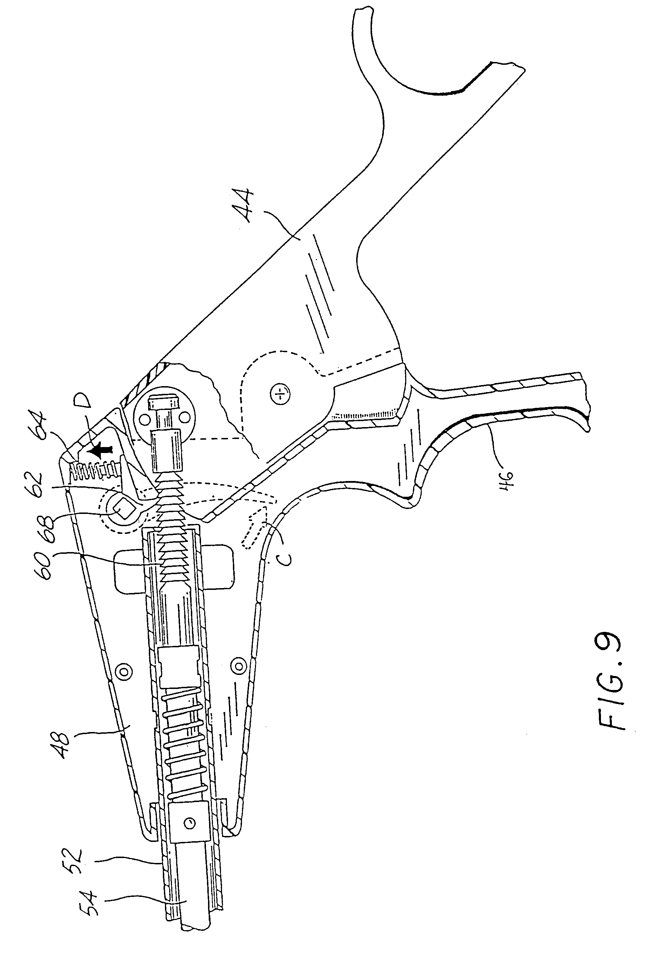

Figure 9 illustrates a cut-away view of the device of Figure 6. Inner rod member 54 includes a rack member 60 which comprises a plurality of circumferential notches cut into rod member 54. The circumferential notches allow for activation of the ratchet mechanism at any orientation of the body assembly 50 due to rotation of rotation knob 56. While it is shown that rod member 54 contains the circumferential notches or indentations, a separate block member may be provided to which rod member 54 is attached to accomplish the same ratcheting principle.

Engaging rack member 60 is a pawl member 62 which is part of ratchet mechanism 58. Pawl member 62 is biased into the engaged position by spring 64, and is pivotable about pivot point 68.

In use, pivoting handle 44 is moved to open and close the jaw members of the tool mechanism (not shown). As pivoting handle 44 moves, pawl member 62 moves along rack member 60 to a desired location for the tool mechanism. To release ratchet mechanism 58, trigger member 66 is moved in the direction of phantom arrow C to overcome the spring force and move the pawl mechanism in the direction of arrow D. Once this mechanism is released, handle 44 is free to move without obstruction. In order to override the ratcheting mechanism, trigger member 66 may be continually depressed in the direction of phantom arrow C so that the pivoting handle 44 may operate freely.

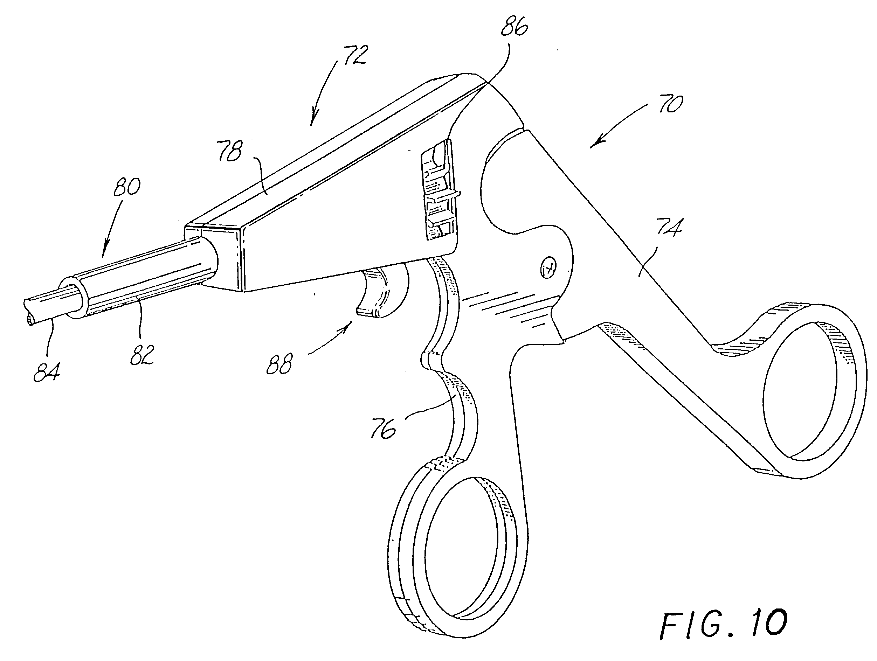

Figure 10 illustrates a third explanatory embodiment of a handle of an endoscopic or laparoscopic surgical instrument employing a ratchet mechanism.

Instrument 70 is similar to devices 10 and 40 above and includes a handle assembly 72 and a body assembly 80. Body assembly 80 terminates in a tool mechanism similar to that described above. Handle assembly 72 includes a pivoting handle 74, a stationary handle 76 and a barrel portion 78. Body assembly 80 includes an outer tube member 82 which is secured to barrel portion 78, and an inner rod member 84 which coaxially passes through outer tube member 82 in sliding arrangement. Inner rod 84 reciprocates within outer tube member 82 upon movement of pivoting handle 74. A rotation knob 86 may be provided, and ratchet mechanism 88 is provided as a trigger grip extending from barrel portion 78.

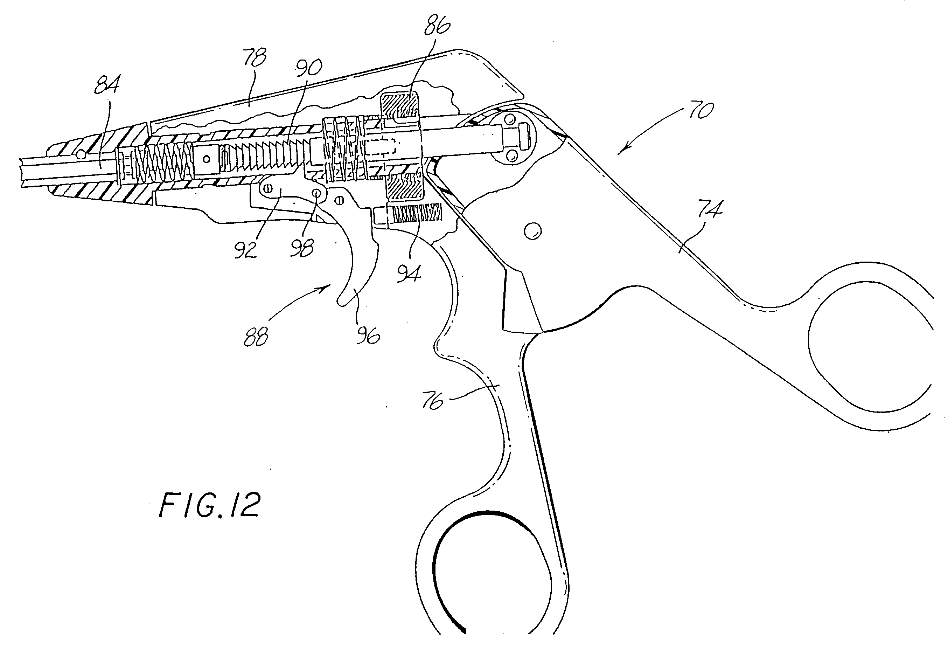

Turning to Figure 12, there is illustrated the ratchet mechanism 88 which is disposed within barrel portion 78. A rack member 90 is provided which comprises a plurality of circumferential notches or indentations in inner rod member 84 which provide for engagement of the ratchet mechanism 88 regardless of the orientation of the tool mechanism due to rotation of rotation knob 86.

In this embodiment, ratchet mechanism 88 essentially comprises an articulated body which is comprised of pawl member 92 and trigger member 96. Trigger member 96 is biased by spring 94 which maintains pawl member 92 in engagement with rack member 90. The articulated body is formed about floating pivot point 98 which joins pawl member 92 with trigger member 96. Both the pawl member 92 and trigger member 96 are each secured at stationary pivot points while floating pivot point 98 allows pawl member 92 to move into and out of engagement with rack member 90.

In use, pivoting handle 74 is moved to set the jaws of the tool mechanism (not shown) to the desired configuration. Spring member 94 biases trigger member 96 forwardly, so that floating pivot point 98 urges pawl member 92 into engagement with rack means 90. To release the ratchet mechanism, trigger member 96 is urged rearwardly against the biasing force of spring 94 so that floating pivot point 98 shifts downwardly to move pawl member 92 out of engagement with rack means 90. Maintaining this rearward depression of trigger member 96 will provide an override for the ratchet mechanism 88, and allow pivoting handle 74 to move freely.

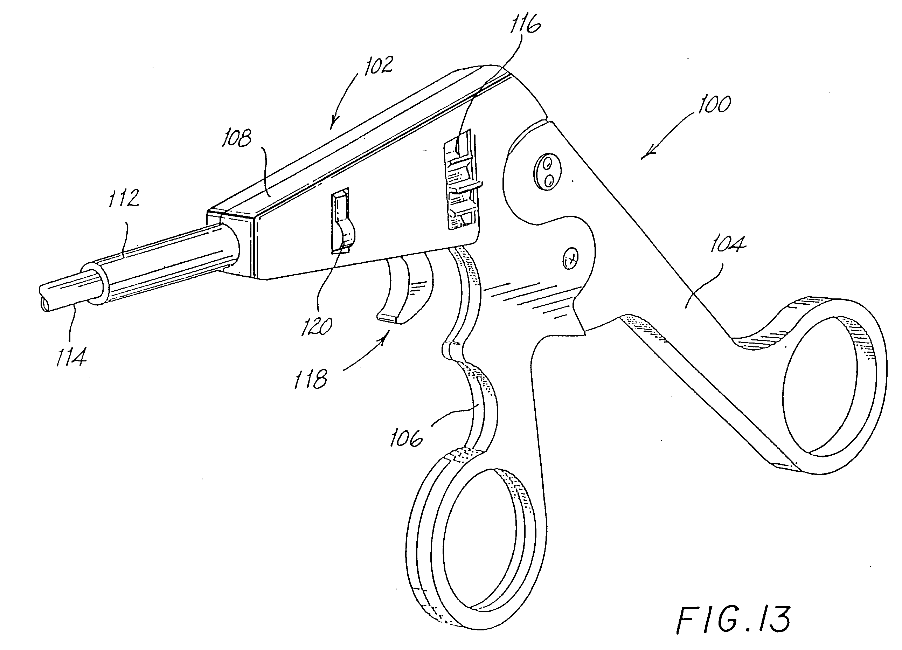

Figure 13 illustrates a fourth explanatory embodiment of a handle of an endoscopic or laparoscopic surgical instrument 100 employing the ratchet mechanism.

Instrument 100 is similar to instruments 10, 40 and 70 above, and includes a handle assembly 102, a body assembly 110, and a ratchet mechanism 118. Handle assembly 102 comprises a pivoting handle 104, a stationary handle 106 and a barrel portion 108, to which body-assembly 110 is secured. Body assembly 110 comprises an outer tubular member 112 and a coaxial inner rod member 114 which slides therein. Outer tube member 112 is secured to barrel portion 108, while inner rod member 114 is secured to pivoting handle 104 and reciprocates within outer tube member 112 upon movement of pivoting handle 104. A rotation knob 116 is provided to adjust the orientation of the tool mechanism (not shown) which is located at the distal end of the body assembly 110. Ratchet mechanism 118 is provided, along with actuation means 120, whose function will be described below.

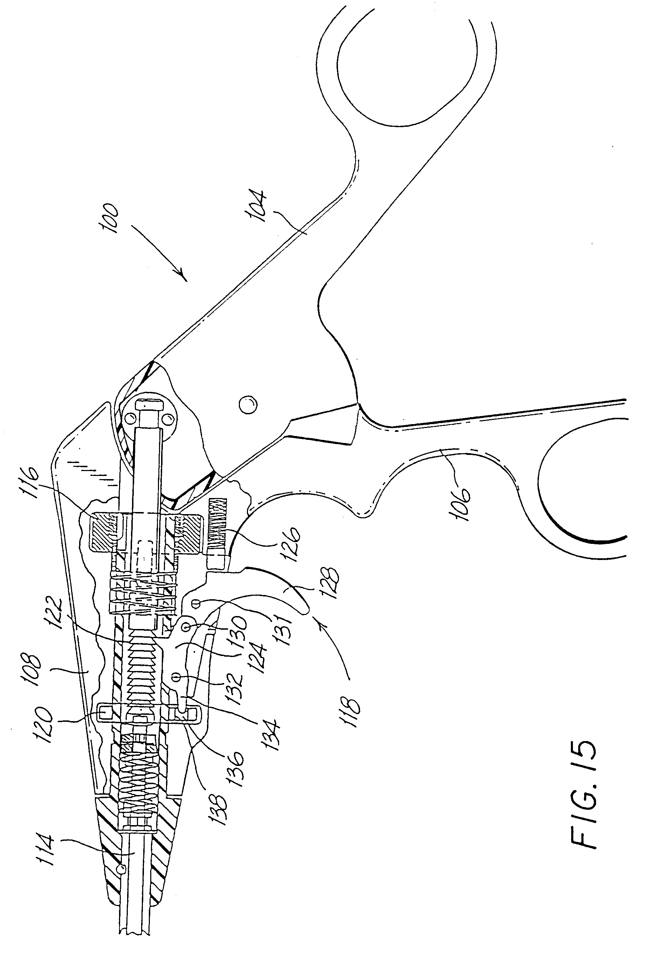

Turning now to Figure 15, there is illustrated a side cut-away view of instrument 100 slowing the ratchet mechanism 118. Inner rod member 114 includes a rack member 122 which comprises a plurality of circumferential notches or indentations which allows for use of the ratchet mechanism 118 regardless of the orientation of the tool mechanism due to rotation of body assembly 110 by rotation knob 116. Ratchet mechanism 118 comprises an articulated body which is formed by pawl member 124, trigger member 128 and a camming member 134 which extends from pawl member 124. Trigger member 128 pivots about a stationary pivot point 131 and is biased in the forward direction by spring 126. Trigger member 128 is joined to pawl member 124 through floating pivot point 130, while pawl member 124 is pivoted further about stationary pivot point 132.

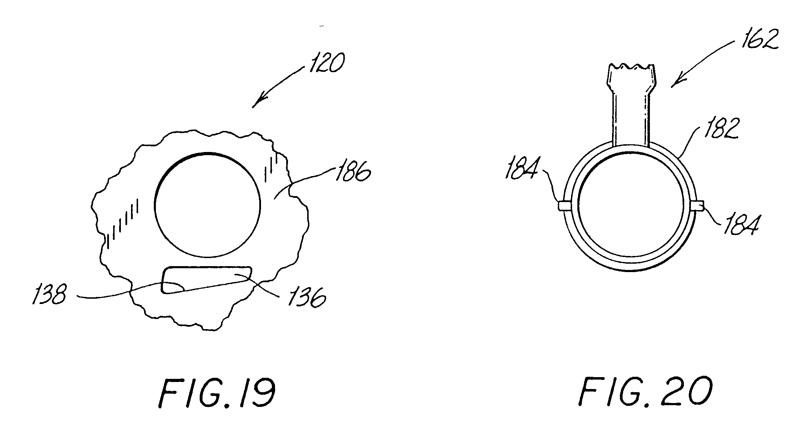

An actuation means 120 is provided, which is best seen in Figure 19. Actuation means 120 comprises a body portion 186 and is provided with a camming slot 136 into which camming member 134 passes. Camming surface 138 engages camming member 134 to urge pawl member 124 into engagement with rack member 122. When actuation means 120 is pushed in a first direction, camming member 134 disengages from camming surface 138 and pawl member 124 disengages from rack member 122. When actuation means 120 is pushed in the opposite direction, camming surface 138 contacts camming member 134 which urges pawl member 124 into engagement with rack member 122. Actuation means 120 functions as a switch to the user to override the ratchet mechanism so that the device 100 may be used in a conventional manner without requiring the user to hold any component of the instrument.

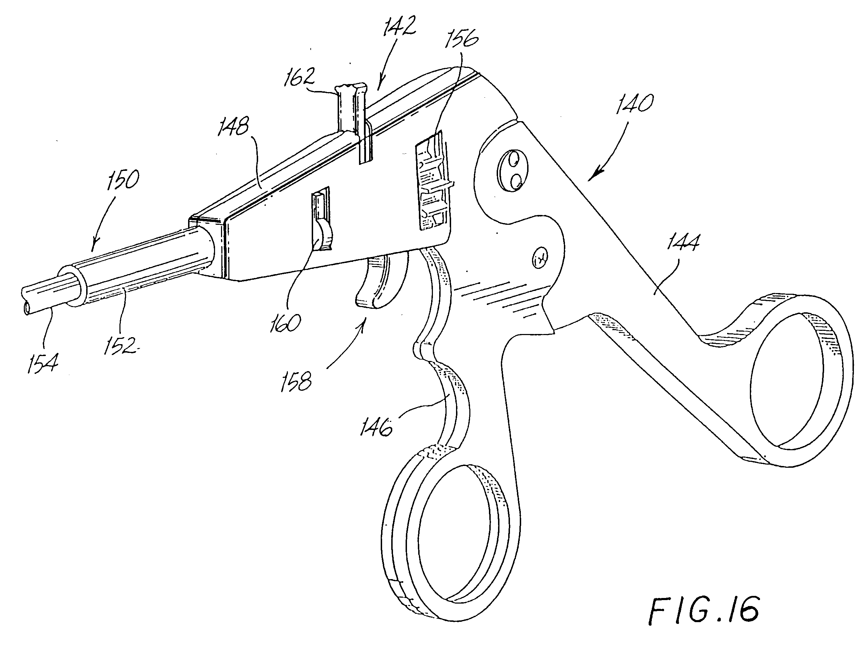

Figure 16 illustrates a fifth explanatory embodiment of a handle of an endoscopic or laparoscopic surgical instrument 140 employing the ratchet mechanism.

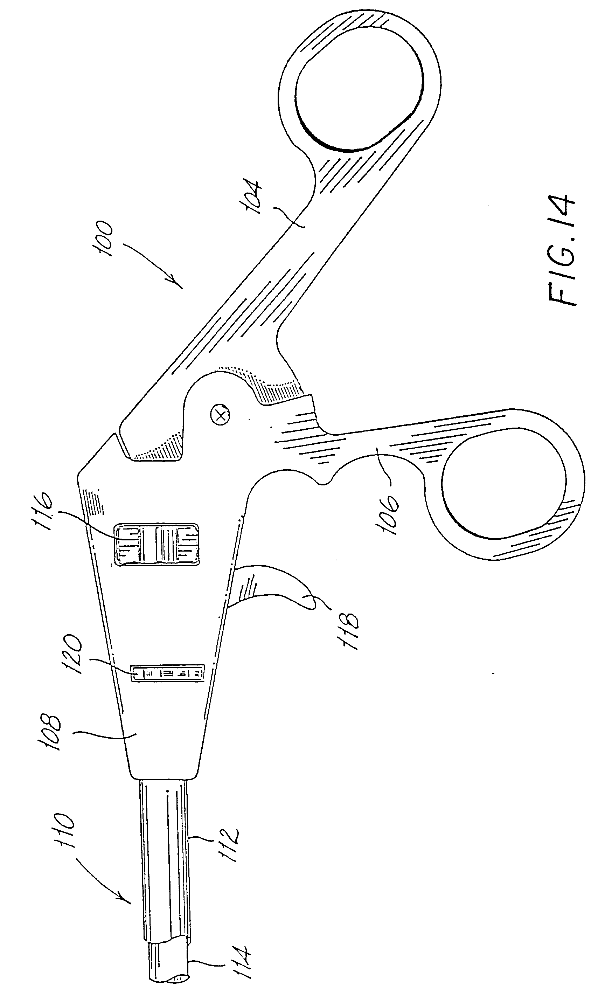

Device 140 is identical to device 100 described above in relation to Figures 13-15, except for the provision of rotation stop means 162.

Instrument 140 comprises handle assembly 142, body assembly 150, and ratchet mechanism 158. Handle assembly 142 comprises pivoting handle 144, stationary handle 146, and barrel portion 148. Body assembly 150 attaches to barrel portion 148 in the manner described above, such that outer tube member 152 is secured to barrel portion 158 while inner rod member 154 slidingly passes through tube member 152 and is secured to pivoting handle 144. Inner rod member 154 reciprocates within outer tube member 152 in response to movement of pivoting handle 144. A rotation knob 156 is provided, along with actuation means 160 which cooperates with ratchet mechanism 158 as described above.

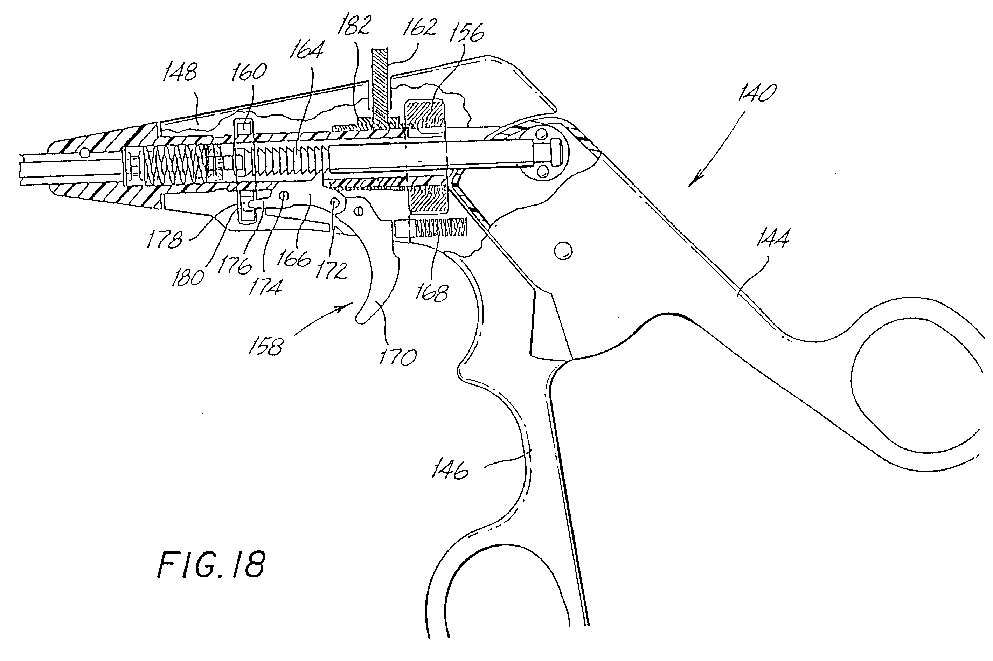

Turning now to Figure 18, and in view of Figure 20, ratchet mechanism 158 and actuation means 160 operate in a manner identical to that described above in reference to Figures 13-15. Stop means 162 is provided having a body portion 182 surrounds outer tube member 152. Rotation of rotation knob 156 allows for various orientations of the tool mechanism (not shown) which is provided at the distal end of body assembly 150. In order to secure body assembly 150 at a particular orientation, stop means 162 is provided which frictionally engages outer tubular member 152 to lock it in place at the desired orientation. The friction force is applied upon rotation of stop means 162 through the provision of guide posts 184 which travel in tracks provided in barrel portion 148. Guide posts 184 provide a torque to body portion 182 which grips outer tubular member 152 to arrest rotational movement at the desired orientation.

Embodiments of the present invention will now be described including novel tool mechanisms in the form of atraumatic jaw mechanisms having jaw members with flexural characteristics. These jaw members are capable of transmitting to users a more accurate "feel" so that they can gauge the amount of force being applied to the captured tissue by the instrument. These jaw members may be fabricated using a unique manufacturing technique which eliminates or substantially reduces the need for elaborate and expensive metal working equipment.

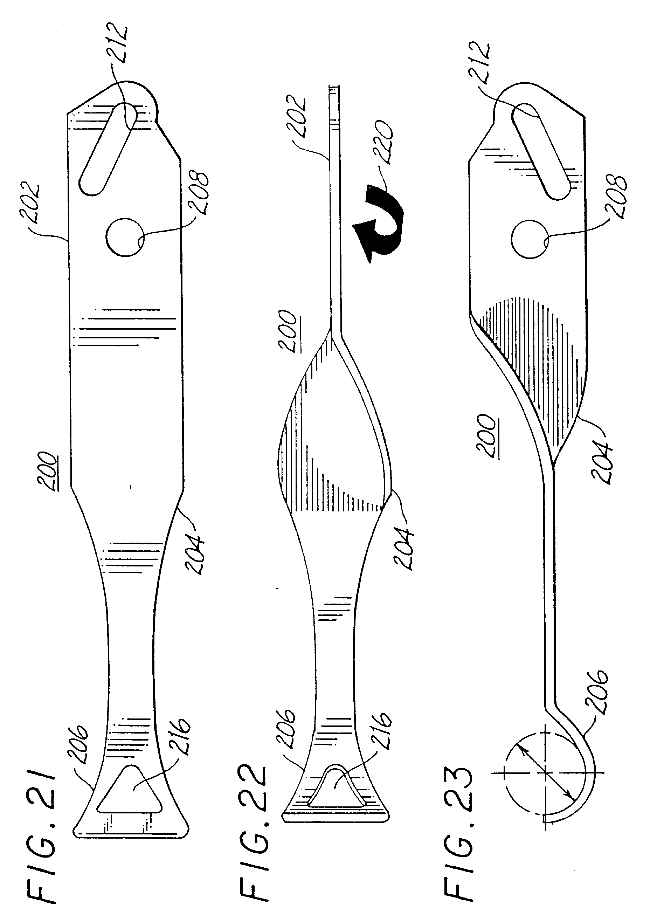

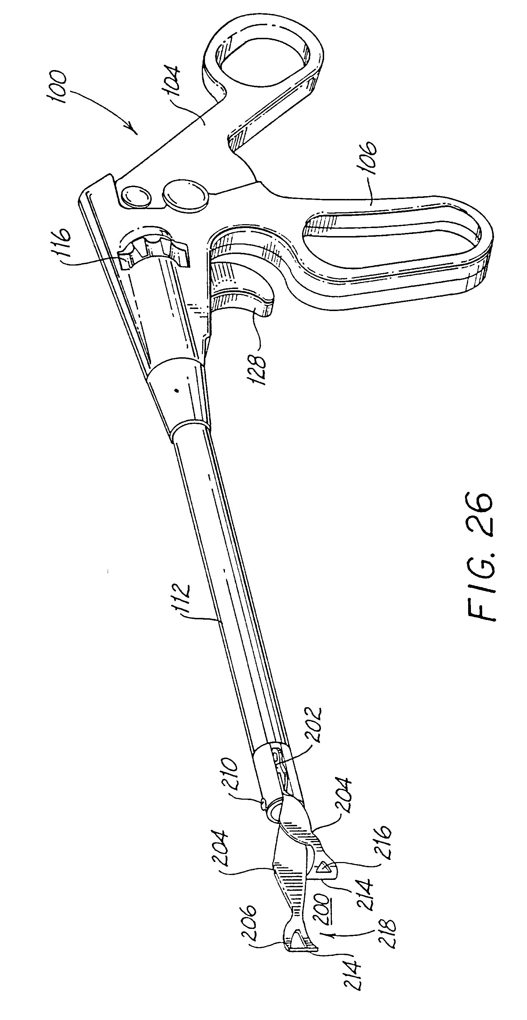

Referring to Figures 21-26 and in particular to Figures 21-23, a novel jaw member is illustrated. This jaw member is shown in the form of a Babcock clamp jaw 200 adapted to capture and hold tubular tissue, preferably without causing traumatic injury. Babcock clamp jaw 200 comprises a proximal portion 202, an intermediate portion 204 and a distal portion 206.

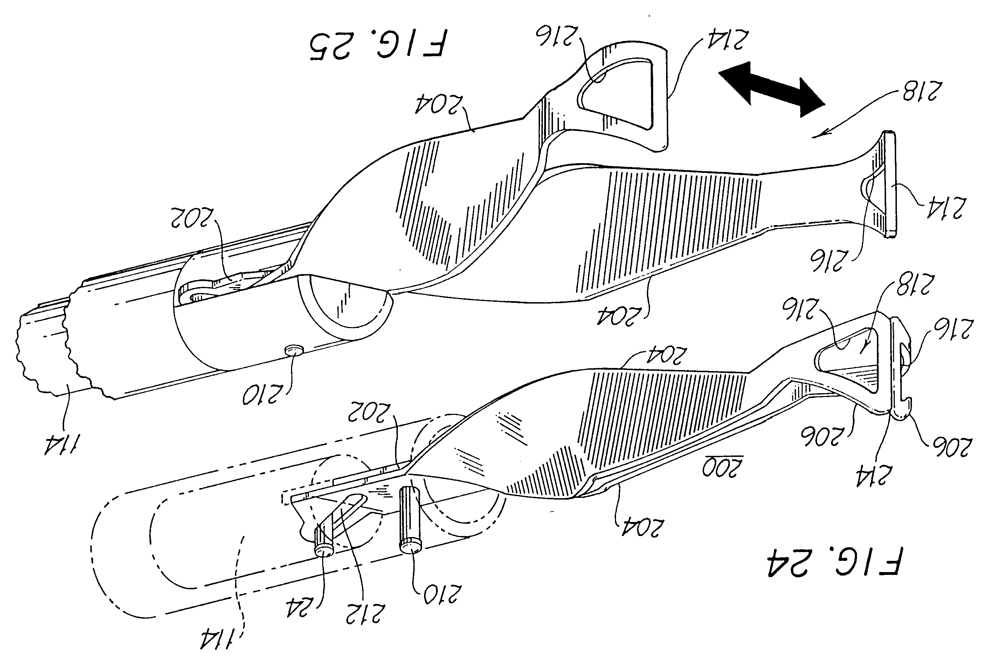

As shown in Figure 21, the proximal portion 202 of the Babcock clamp jaw 200 is provided with a pivot bore 208 transversely formed therein for receiving a pivot pin 210 (Figure 24) to interconnect a pair of jaw members. Proximal portion 202 further includes means for moving the interconnected jaw members pivotally relative to one another. In the embodiment shown in Figures 21-26, this structure comprises a diagonal camming slot 212 formed in the proximal portion 202, which slot receives a camming pin 24 connected to and controlled by inner rod member 114. Note that the present novel jaw structure is described in the context of a surgical instrument in accordance with the embodiment of Figure 15 discussed hereinabove. However, the jaw structure may be equally incorporated into other surgical instrument configurations including the alternate explanatory embodiments discussed herein.

The distal portion 206 of the Babcock clamp jaw 200 includes a contact surface 214 and a tissue expansion bore 216 formed therein. Tissue expansion bore 216 also provides a fluid passage for fluids associated with grasped tissue. The distal portion is formed into a substantially semicircular shape defining a tissue capturing space 218 on an interior surface thereof.

Intermediate portion 204 is disposed between proximal portion 202 and distal portion 206 and permits the distal portion 206 to flex transversely with respect to proximal portion 202 when the contact surface 214 of two opposing clamp jaw members contact or when an enlarged tissue structure is contained within tissue capturing space 218. In the embodiment of Figures 21-26, the distal portions 206 of clamp jaw member 200 are precambered inward such that as the opposing claim jaws are approximated, the contact surface 214 of the respective distal portions 206 come into abutment first. This configuration allows the intermediate portion 204 to flex or bend thus transmitting a feel to the user of the force being applied.

The Babcock clamp jaws 200 may advantageously be formed in a novel manner from a single sheet of material. Preferably, this material is relatively strong and malleable with good flexural characteristics, e.g. Series 302 or 304 stainless steel.

To form the jaws, the basic shape of the jaw is stamped or formed out of a single flat sheet of material. Thereafter, any desired bores or slots may be formed as desired. Once the basic flat shape is present, the distal end of the flattened workpiece is twisted or bent so that the distal end is disposed in a predetermined angular orientation with respect to the proximal portion of the workpiece. In the embodiment of Figures 21-26, the distal portion 206 is twisted in the direction of arrow 220 (Figure 22) until the distal portion 206 is disposed in a plane approximately 90° transverse to the plane of the proximal portion 202.

Once the workpiece is bent into the correct shape, the distal portion 206 of the jaw member 200 is formed into its final semicircular shape using, for example, a dowel or other forming tool of a predetermined shape. See Figure 23. The distal portion may also be precambered to provide a larger range of flexibility for the jaw members.

The embodiment of Figures 21-26 demonstrates jaw members wherein the distal portions thereof are each bent in the same transverse c!rection relative to the respective proximal portions. This advantageous configuration permits the jaws, when assembled together to overlap and minimize visual obstruction of the tissue capturing area 218.

Referring to Figure 24, opposing Babcock clamp jaws 200 are disposed in a closed approximated position with intermediate portions 204 overlapping and contact surfaces 214 in abutment. This position facilitates insertion of the instrument 222 through a cannula (not shown). Once in place within the endoscopic operative site, the ratcheting mechanism 118 (Figure 15) is released by means of trigger 128 allowing Babcock clamp jaws 200 to spring open in preparation for tissue capture. The subject tissue (not shown) is maneuvered into the tissue capturing space 218 and the jaws 200 are reapproximated using pivoting handle 104 and stationary handle 106 to retract coaxial inner rod member 114 relative to outer tube member 112. As the contact surfaces 214 of the distal portions 206 abut, the user may effectively gauge the application of more force to the captured tissue as the distal portion of the jaws flex via the intermediate portion relative to the proximal portion.

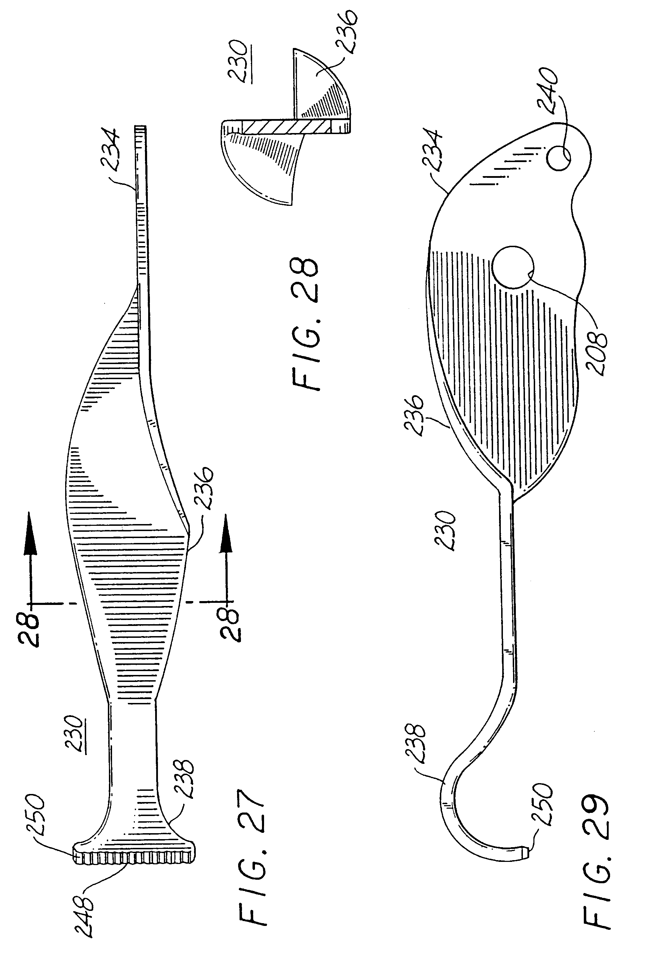

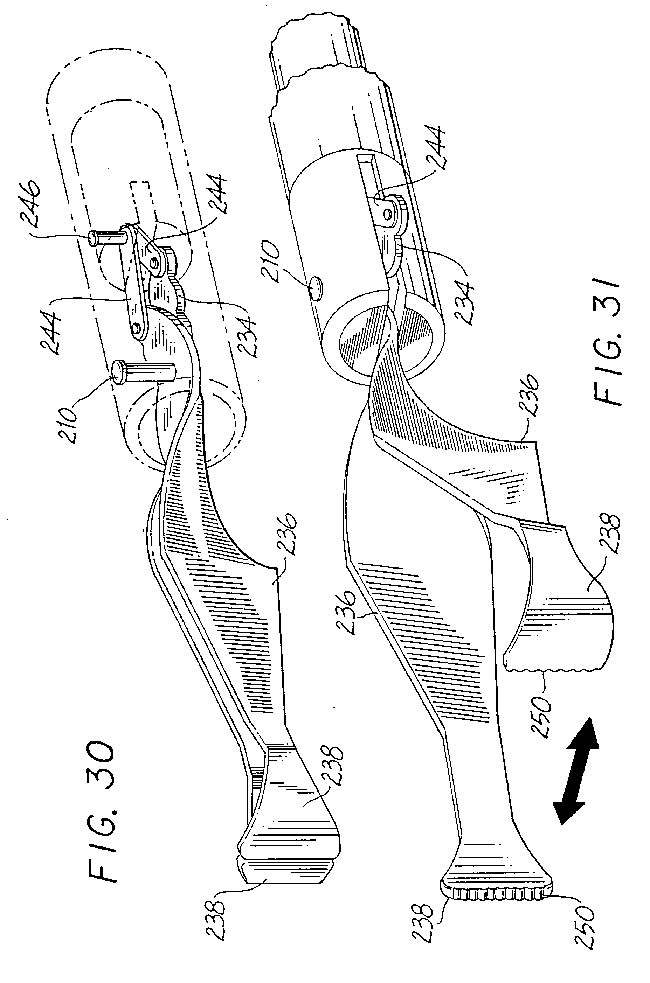

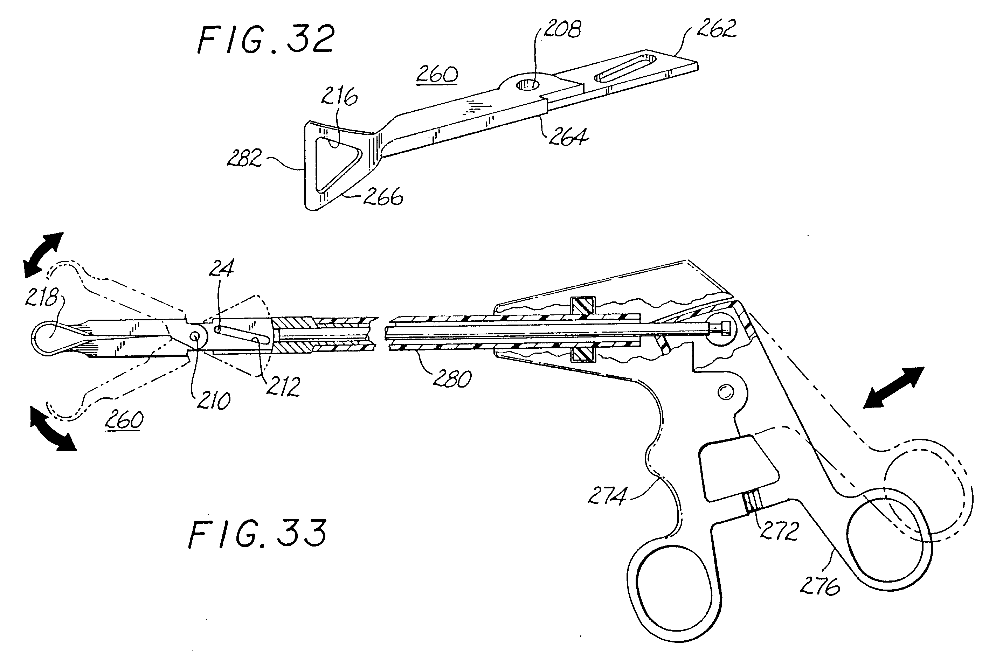

Figures 27-31 illustrate a further embodiment of Babcock clamp jaws 230 in accordance with the present invention. These jaws 230 are substantially the same as Babcock clamp jaws 200 discussed above with the exception of the linkage means 232 used to connect the jaws 200 to the inner rod 114 and activate the jaws to pivotally move them between an open position (Figure 31) and a closed position (Figure 30).

The jaws 230 include a proximal portion 234, an intermediate portion 236 and a distal portion 238. The proximal portion 234 includes a pivot bore 208 for receiving pivot pin 210 and a link pin bore 240 for receiving link pin 242 to connect the jaw 230 to articulating link 244. Links 244 of opposing jaws 230 are connected to coaxial inner rod member 114 at pin 246, and serve to drive the jaws 230 between an open and closed position as the rod member 114 is moved coaxially with respect to outer tube member 112.

The intermediate portion 236 is substantially the same as intermediate portion 204 of Figures 21-26 and has flexural characteristics which allow distal portion 238 to flex relative to proximal portion 234.

The distal portion 238 is solid and terminates in a more aggressive contact surface 248 provided with interdigitating teeth 250. As in the previous embodiment, the distal portion is precambered inwardly so that as the jaws 230 are approximated, the contact surface 248 of respective opposing jaws contact first, leaving a space separating said intermediate portions 236 to allow for flexing of the jaws 230 relative to one another.

Turning now to Figures 32 and 33, there is illustrated a third configuration of the Babcock clamp jaw in accordance with the present invention. The clamp jaw 260 includes a proximal portion 262, an intermediate portion 264 and a distal portion 266. The proximal and distal portions of this embodiment of the Babcock clamp jaw 260 are formed in substantially the same configuration as the Babcock clamp jaw 200 disclosed above. The intermediate portion 264 is formed with the bend 268 located almost directly adjacent the near end of the distal portion 266 such that only a relatively small percentage of the jaw structure is disposed in the distal portion in a plane perpendicular to the plane of the proximal portion 262. This configuration allows for a longer intermediate portion 264 while maintaining adequate control and flexural characteristics of the jaw 260. Also, the distal portions 266 of opposing jaw members 260 are formed or bent in correspondingly opposite transverse directions and do not substantially overlap as in jaws 200 and 230.

Figure 33 shows an instrument 270 incorporating jaws 260 in conjunction with an external ratchet 272 disposed between the stationary handle 274 and the pivoting handle 276. In the open position (shown in phantom) camming pin 24 disposed in coaxial inner rod 278 is in its distal most position camming opposing jaws 260 apart. Compression of handles 274, 276 together causes inner rod 278 to retract relative to outer tube 280 camming the jaws 260 together such that contacting surfaces 282 of the distal portion 266 of jaw 260 come in contact. Further compression of the handles 274, 276 causes the distal portion 266 to flex relative to the proximal portion 262 through intermediate portion 264. The external ratchet 272 permits the jaws 260 to be locked in preselected degrees of closure and/or compression.

While the above embodiments have been described in the context of a Babcock clamp jaw, other jaw structure may advantageously be formed in this fashion and in these configurations. Examples of other appropriate jaw structures include graspers, dissectors forceps etc.

The foregoing describes a novel endoscopic or laparoscopic surgical device which incorporates many features necessary for endoscopic or laparoscopic surgical procedures, and provides a lightweight and easy to us device which may be operated with one hand. The device may include an internal ratcheting mechanism located preferably within the barrel of the handle mechanism which provides for incremental positioning of the tool mechanism for performing the surgical procedure. The device is simple to manufacture. The device is a high precision instrument in which many moving parts normally associated with such a device are eliminated, thus reducing instances of mechanical failure requiring expensive repair or ultimate destruction of the instrument.

An endoscopic or laparoscopic surgical instrument usually comprises a handle assembly, an elongated body assembly and a tool mechanism. A ratchet mechanism may be attached within the barrel portion of the handle assembly. The handle assembly includes a stationary handle and pivoting handle, attached to the barrel portion, and the body assembly is attached to the barrel portion and extends therefrom. The body assembly consists of an outer tubular member and an inner rod member which coaxially passes within the outer tubular member. The rod member is attached to the pivoting handle, while the tube member is secured in a conventional manner to the barrel portion which extends into the stationary handle. As the pivoting handle moves, the rod member slidably reciprocates within the outer tube member.

Attached to a distal end of the body assembly is the tool mechanism which opens and closes in response to movement of the pivoting handle in relation to the stationary handle. The tool mechanism may comprise a jaw mechanism with a pair of jaw members wherein one or both jaw members open and close to perform various endoscopic or laparoscopic surgical procedures. The tool mechanism includes, but is not limited to, a scissor device, a dissecting device, a grasping device, a retractor device, and like mechanisms.

The instrument according to the invention includes novel atraumatic jaw mechanisms having flexural characteristics which serve to better transmit to the user a more accurate gauge of the force being applied to the captured tissue. This is accomplished by fabricating the jaw members of a material or in a configuration which permits the jaw members to flex and allow the user to gauge the amount of force applied to the tissue by the jaw mechanism. This flexural ability reduces the likelihood of unintentional traumatic injury, particularly in reduced visibility, endoscopic or laparoscopic procedures. In particularly advantageous embodiments, the jaw members are fabricated using a unique manufacturing technique which eliminates the need for elaborate and expensive metal working equipment. This technique includes fabricating the individual jaw members from a single sheet of malleable material and twisting or bending the jaw members into a predetermined position.

The instrument also includes the provision of a second pivot point on the pivoting handle, to which the inner rod member is attached. As the handle pivots, the second pivot point rotates to allow the inner rod to move longitudinally in the outer tube with minimal radial deflection. This feature reduces the radial wear on the inner rod and prevents weakening of the structure during long term use. In addition, it allows for a reduction of the required internal spacing between the outer tube and inner rod to result in a more compact and streamlined instrument. Furthermore, unwanted torquing forces are eliminated at the pivot point thus minimizing the possibility of mechanical breakdown of the instrument at the connection between the pivoting handle and the movable inner rod.

The instrument may include the provision of a rotatable knob on the outer tubular member to allow the body assembly and tool mechanism to rotate to position the jaws at desired angles to the longitudinal axis during the surgical procedure. Preferably, the rotatable knob is secured to the outer tube and positioned in a slot which passes through the barrel portion of the stationary handle, so that a surgeon may rotate the knob, and consequently the body assembly and jaw mechanism, through the use of his thumb while he is holding the stationary handle with his fingers. This frees the surgeon's other hand to simultaneously operate another instrument during surgery.

Another feature of the instrument is the provision of a ratchet mechanism located internally within the barrel of the handle assembly to provide for incremental movement of the jaw mechanism. Since it is located internally within the barrel portion of the handle assembly, it is not subjected to environmental conditions which may result in clogging or damage to the ratchet mechanism during handling and storage. Furthermore, the ratchet mechanism described herein provides for simple handling and maneuvering during the surgical procedure and allows the surgeon to operate the device with one hand, thus freeing his other hand for performing other functions during the surgical procedure.

The ratchet mechanism includes a trigger mechanism for engaging and disengaging the ratchet feature. In a first embodiment, a rack member is provided on the surface of the pivoting handle which engages the pawl arm of the trigger portion of the ratchet mechanism. The pawl arm is biased by a leaf spring member which maintains the pawl arm in contact with the rack member. The trigger member, when depressed, overcomes the force of the leaf spring and pivots the pawl arm away from the rack member to release the ratchet mechanism. If the trigger mechanism is continually pressed, the ratchet mechanism is overridden and the device functions as a conventional surgical instrument. The trigger mechanism is preferably positioned on the barrel portion of the stationary handle member. The rack member consists of a plurality of indentations or notches into which the pawl arm fits to secure the handles in incremental positions during operation of the tool mechanism.

A second embodiment of the ratchet mechanism provides the trigger mechanism positioned on the stationary handle at the barrel portion and includes a pawl arm which engages a rack member which is constructed integral with the inner rod member of the body assembly. The rack member may comprise a plurality of indentations cut into the rod member which engage the pawl arm of the trigger mechanism. The trigger mechanism is spring biased so that the pawl arm is continually engaged with the rack member. Constant depression of the trigger mechanism overrides the ratchet mechanism and the handles may be operated as in a conventional tool.

Alternately, the indentations may be part of a block device which is secured to the rod member and provided with the plurality of indentations or notches to engage the pawl arm. Preferably, however, the notches or indentations are constructed integral with the rod member, and in a preferred embodiment are provided as a series of circumferential notches about the rod member. This allows for the provision of a rotatable body assembly through the use of a rotation knob which provides 360°, or any portion thereof, rotation of the body assembly to rotate the jaws of the tool mechanism to desired angles along the longitudinal axis of the instrument during the surgical procedure. Accordingly, the ratchet mechanism may operate at any orientation of the jaw members.

In order to provide a complete override feature of the ratchet mechanism, the instrument may include an actuator device which cooperates with the trigger mechanism to provide an on/off mechanism for the ratchet feature. In this embodiment, the trigger mechanism includes an articulated body portion having a projection or finger-like member which acts as a camming member to engage the actuator means. The actuator means essentially comprises a pivotable camming member having a slot into which the finger-like projection extends. When pivoted in a first direction, the camming slot engages the camming member of the articulated body and causes the body to pivot into engagement with the circumferential rack disposed on the inner rod member. When the actuator means is pivoted in a second direction, the camming slot is of such a configuration so as to disengage with the camming member of the articulated body which causes the pawl arm to fall out of engagement with the rack means of the inner rod member. In this embodiment, the trigger mechanism is also spring biased so that when the actuator means is in the "on" position, the pawl arm is biased into engagement with the rack means.

A further optional feature is the provision of a stop mechanism to arrest rotation of the body assembly. The stop mechanism is provided in conjunction with the rotation knob and allows the surgeon to lock the body assembly at a particular orientation during rotation. The lock mechanism is provided on the barrel portion of the handle assembly and is positioned so that the surgeon may activate the lock mechanism with a single hand.

The instrument may also feature a connection port to provide the device with electocautery capabilities. A connection port allows for the connection of a suitable jack member tube inserted into the device. The outer tube of the body assembly is provided with electrical insulation, preferably heat shrink tubing, which extends a substantial portion of the length of the outer tube. In this embodiment, the handle is molded of plastic material to provide electrical insulation for the user.

In the preferred embodiment, all the above features are incorporated into a single endoscopic and laparoscopic surgical instrument, so that the instrument has electrocautery, rotational, and ratcheting capabilities. However, the instrument is usually constructed with at least the ratcheting capabilities to provide for incremental adjustment of the tool mechanism during a surgical procedure.

## Claims

1. A surgical instrument comprising:
a pair of jaws (200);
means (208) for pivotally mounting said jaws in cooperative alignment; and
means (100) for selectively pivoting said jaws,
characterized in that each jaw is formed from a single sheet of material having a proximal (202), distal (206) and intermediate portion (204), said jaws being twisted in said intermediate portion (204) such that said distal portion (206) is substantially perpendicular to said proximal portion (202);
wherein said jaws (200) are precambered such that when they are pivoted to a closed position, said jaws contact each other at respective contact surfaces (214, 248) of the distal portions thereof before said intermediate portions come into contact.

2. An instrument as claimed in claim 1 wherein said distal portions (206) of said jaws each have a substantially semicircular shape such that, when said jaws are pivoted to a closed position, said distal portions of said jaws provide a substantially circular opening (218) adapted for grasping tubular tissue.

3. An instrument as claimed in claim 1 or 2 wherein the distal portions of said first and second jaws are flexible such that, upon further movement to close said jaws after first contact of said distal portions, said distal portions (206) flex relative to said proximal portions (202).

4. An instrument as claimed in any one of the preceding claims wherein a tissue-engaging surface (248) of the distal portions of said jaws (200) comprises a plurality of teeth (250) formed on said surface.

5. An instrument as claimed in any one of the preceding claims, further comprising a handle assembly (100) and a body assembly (112) extending from said handle assembly (100), said jaws (200) extending from a distal end of said body assembly and moveable in response to movement of said handle assembly (100).

6. An instrument according to claim 5 wherein said body assembly comprises an elongate tubular member (112).

7. An instrument as claimed in claim 5 or 6 further comprises means (116) for rotating said body assembly (112) to orient said jaws.

8. An instrument as claimed in claim 7 wherein said means for rotating said body assembly comprises a knob member (116) circumferentially disposed about said body assembly and protruding radially outward from said body assembly through slots in said handle assembly (100).

9. An instrument as claimed in claim 8 wherein said means for rotating said body assembly further comprises a stop means for arresting rotational movement of said body assembly.

10. An instrument as claimed in any one of claims 5 to 9 further comprising a ratchet mechanism (118) positioned within said handle assembly, said ratchet mechanism engaging said body assembly to provide incremental positioning of said jaws members between said open and closed positions.

11. An instrument as claimed in any one of the preceding claims wherein the proximal portions of said jaws are provided with camming slots (212) for engaging a bearing surface on a linkage means (24, 114).

12. An instrument as claimed in claim 11 wherein the proximal portions of said jaws include hinged connection means for connecting said jaws and said linkage means (24, 114).

## Patentansprüche

1. Chirurgisches Instrument, umfassend:
ein Paar von Klemmbacken (200);
eine Einrichtung (208), um die Klemmbacken in einer zusammenwirkenden Ausrichtung schwenkbar zu befestigen; und
eine Einrichtung (100), um selektiv die Klemmbacken zu verschwenken,
dadurch **gekennzeichnet,** daß
jede Klemmbacke aus einem einzelnen Materialbogen geformt ist mit einem proximalen (202), distalen (206) und Zwischenbereich (204), wobei die Klemmbacken in dem Zwischenbereich (204) so verdreht sind, daß der distale Bereich (206) im wesentlichen senkrecht zu dem proximalen Bereich (202) ist;
wobei die Klemmbacken (200) so vorgewölbt sind, daß, wenn sie in eine geschlossene Position verschwenkt werden, die Klemmbacken einander an entsprechenden Kontaktoberflächen (214, 248) der distalen Bereiche derselben kontaktieren, bevor die Zwischenbereiche in Kontakt treten.

2. Instrument gemäß Anspruch 1, wobei die distalen Bereiche (206) der Klemmbacken alle eine im wesentlichen halbkreisförmige Form besitzen, so daß, wenn die Klemmbacken in eine geschlossene Position verschwenkt werden, die distalen Bereiche der Klemmbacken eine im wesentlichen kreisförmige Öffnung (218) vorsehen, die zum Greifen von röhrenförmigem Gewebe angepaßt ist.

3. Instrument gemäß Anspruch 1 oder 2, wobei die distalen Bereiche der ersten und zweiten Klemmbacken so biegsam sind, daß bei einer weiteren Bewegung zum Schließen der Klemmbacken nach dem ersten Kontakt der distalen Bereiche sich die distalen Bereiche (206) relativ zu den proximalen Bereichen (202) verbiegen.

4. Instrument gemäß einem der vorhergehenden Ansprüche, wobei eine Gewebeeingriffsoberfläche (248) der distalen Bereiche der Klemmbacken (200) eine Mehrzahl von Zähnen (250) umfassen, die auf der Oberfläche gebildet sind.

5. Instrument gemäß einem der vorhergehenden Ansprüche, weiter umfassend einen Griffaufbau (100) und einen Körperaufbau (112), der sich von dem Griffaufbau (100) erstreckt, wobei sich die Klemmbacken (200) von einem distalen Ende des Körperaufbaus erstrecken und in Antwort auf eine Bewegung des Griffaufbaus (100) bewegbar sind.

6. Instrument gemäß Anspruch 5, wobei der Griffaufbau ein langgestecktes röhrenförmiges Element (112) umfaßt.

7. Instrument gemäß Anspruch 5 oder 6, weiter umfassend eine Einrichtung (116), um den Körperaufbau (112) zu drehen, um die Klemmbacken auszurichten.

8. Instrument gemäß Anspruch 7, wobei die Einrichtung zum Drehen des Körperaufbaus umfaßt ein Knopfelement (116), das in Umfangsrichtung um den Körperaufbau angeordnet ist und in radialer Richtung nach außen von dem Körperaufbau durch Schlitze in dem Griffaufbau (100) vorsteht.

9. Instrument gemäß Anspruch 8, wobei die Einrichtung zum Drehen des Körperaufbaus weiter umfaßt eine Anschlageinrichtung, um die Drehbewegung des Körperaufbaus anzuhalten.

10. Instrument gemäß einem der Ansprüche 5 bis 9, weiter umfassend einen Ratschenmechanismus (118), der innerhalb des Griffaufbaus positioniert ist, wobei der Ratschenmechanismus in den Körperaufbau einrückt, um für ein schrittweises Positionieren der Klemmbackenelemente zwischen der offenen und geschlossenen Position zu sorgen.

11. Instrument gemäß einem der vorhergehenden Ansprüche, wobei die proximalen Bereiche der Klemmbacken mit Kurvenschlitzen (212) versehen sind, um in Eingriff zu treten mit einer Lageroberfläche auf einer Verbindungseinrichtung (24, 114).

12. Instrument gemäß Anspruch 11, wobei die proximalen Bereiche der Klemmbacken eine Gelenkverbindungseinrichtung zum Verbinden der Klemmbacken und der Verbindungseinrichtung (24, 114) umfassen.

## Revendications

1. Instrument chirurgical comportant :
une paire de mâchoires (200) ;
un moyen (208) pour monter de manière pivotante lesdites mâchoires suivant un alignement de coopération ; et
un moyen (100) pour faire pivoter sélectivement lesdites mâchoires,
caractérisé en ce que chaque mâchoire est formée à partir d'une seule feuille de matériau comportant des portions proximale (202), distale (206) et intermédiaire (204), lesdites mâchoires étant tordues dans ladite portion intermédiaire (204) de façon que ladite portion distale (206) soit sensiblement perpendiculaire à ladite portion proximale (202) ; où lesdites mâchoires (200) sont cambrées préalablement de façon que lorsqu'elles sont pivotées en une position fermée, lesdites mâchoires viennent en contact l'une avec l'autre à des surfaces de contact respectives (214, 248) de leurs portions distales avant que lesdites portions intermédiaires viennent en contact.

2. Instrument selon la revendication 1, où lesdites portions distales (206) desdites mâchoires ont chacune une forme sensiblement semi-circulaire de telle sorte que lorsque lesdites mâchoires sont pivotées en une position fermée, lesdites portions distales desdites mâchoires réalisent une ouverture (218) sensiblement circulaire apte à saisir le tissu tubulaire.

3. Instrument selon la revendication 1 ou 2, où les portions distales desdites première et seconde mâchoires sont flexibles de telle sorte que lors d'un mouvement ultérieur pour fermer lesdites mâchoires après un premier contact desdites portions distales, lesdites portions distales (206) fléchissent relativement auxdites portions proximales (202).

4. Instrument selon l'une des revendications précédentes, où une surface de mise en prise avec le tissu (248) des portions distales desdites mâchoires (200) comporte une pluralité de dents (250) formées sur ladite surface.

5. Instrument selon l'une des revendications précédentes, comportant en outre un ensemble de poignée (100) et un ensemble de corps (112) s'étendant depuis ledit ensemble de poignée (100), lesdites mâchoires (200) s'étendant d'une extrémité distale dudit ensemble de corps et sont déplaçables en réponse au déplacement dudit ensemble de poignée (100).

6. Instrument selon la revendication 5, où ledit ensemble de corps comporte un élément tubulaire oblong (112).

7. Instrument selon la revendication 5 ou 6, comportant en outre un moyen (116) pour faire tourner ledit ensemble de corps (112) afin d'orienter lesdites mâchoires.

8. Instrument selon la revendication 7, où ledit moyen pour faire tourner ledit ensemble de corps comporte un élément de bouton (116) disposé circonférentiellement autour dudit ensemble de corps et faisant saillie radialement vers l'extérieur depuis ledit ensemble de corps à travers des fentes dans ledit ensemble de poignée (100).

9. Instrument selon la revendication 8, où ledit moyen pour faire tourner ledit ensemble de corps comporte en outre un moyen d'arrêt pour arrêter le mouvement de rotation dudit ensemble de corps.

10. Instrument selon l'une des revendications 5 à 9, comportant en outre un mécanisme à rochet (118) positionné dans ledit ensemble de poignée, ledit mécanisme à rochet étant mis en prise avec ledit ensemble de corps pour réaliser un positionnement incrémentiel desdits éléments de mâchoire entre lesdites positions ouverte et fermée.

11. Instrument selon l'une des revendications précédentes, où les portions proximales desdites mâchoires présentent des fentes (212) de réception de came pour être mises en prise avec une surface de support sur un moyen de liaison (24, 114).

12. Instrument selon la revendication 11, où les portions proximales desdites mâchoires comportent des moyens de connexion articulés pour relier lesdites mâchoires et lesdits moyen de liaison (24, 114).
